# EUROPEAN PATENT SPECIFICATION

(11) **EP 2 819 986 B1**
(45) Date of publication and mention of the grant of the patent: **31.05.2017**
(21) Application number: 13755346.7
(22) Date of filing: 20.02.2013
(51) Int. Cl.: C07C 67/00, B01J 29/40, C07C 1/20, C07C 41/01, C07C 67/39, C07C 43/06, C07C 69/716, C13K 1/02, C07G 1/00

(54) **CATALYTIC CONVERSION OF LIGNO-CELLULOSIC BIOMASS INTO FUELS AND CHEMICALS**
KATALYTISCHE UMWANDLUNG VON LIGNOCELLULOSE-BIOMASSE IN KRAFTSTOFFE UND CHEMISCHE STOFFE
CONVERSION CATALYTIQUE DE BIOMASSE LIGNOCELLULOSIQUE EN COMBUSTIBLE ET EN PRODUITS CHIMIQUES

(30) Priority: 29.02.2012 US 201261604726 P
(43) Date of publication of application: 07.01.2015
(73) Proprietor: AC3B Technologies Ltd., Ville Saint-Laurent, QC H4R 1S2 (CA)
(72) Inventor: LE VAN MAO, Raymond, Ville Saint-Laurent QC H4R 1S2 (CA)
(74) Representative: Hart-Davis, Jason
(86) International application number: PCT/CA2013/050130
(87) International publication number: WO 2013/127006

(56) References cited:
- WO-A1-2009/156842
- LE VAN MAO ET AL.: 'NEW PROCESS FOR THE ACID-CATALYZED CONVERSION OF CELLULOSIC BIOMASS (AC3B) INTO ALKYL LEVULINATES AND OTHER ESTERS USING A UNIQUE ONE-POT SYSTEM OF REACTION ANDPRODUCT EXTRACTION' CATAL. LETT. vol. 141, 2011, pages 271 - 276, XP019882943
- XIANG ET AL.: 'OXIDATIVE CRACKING OF PRECIPITATED HARDWOOD LIGNIN BY HYDROGEN PEROXIDE' APPL. BIOCHEM. BIOTECHNOL. vol. 84-86, 2000, pages 153 - 162, XP055160316
- LE VAN MAO ET AL.: 'AC3B TECHNOLOGY FOR DIRECT LIQUEFACTION OF LIGNOCELLULOSIC BIOMASS: NEW CONCEPTSOF COUPLING AND DECOUPLING OF CATALYTIC/CHEMICAL REACTIONS FOR OBTAINING A VERY HIGH OVERALL PERFORMANCE' CATAL. LETT. vol. 142, 27 April 2012, pages 667 - 675, XP035059430

## Description

### FIELD OF THE INVENTION

This invention relates to a process for producing ethyl esters and hydrocarbons from ligno-cellulosic biomass materials.

### BACKGROUND OF THE INVENTION

Ligno-cellulosic biomass material designates materials such as wood, forestry, paper-making or cardboard-making residues, agricultural residues, municipal wastes and perennial grasses. Paper pulp used in the pulp and paper industry is an example of cellulosic material albeit poor in lignin content. Other examples of ligno-cellulosic biomass materials are: wood chips (jack pine, spruce, etc), switch grass or logging operation residues. Ligno-cellulosic biomass material will vary in their composition in cellulose, hemicellulose, lignin and other species. Typically, ligno-cellulosic materials contain as main components: cellulose (about 35-50 wt %), hemicellulose (about 23-30 wt %) and lignin (about 15-32 wt %).

Cellulose is made up of crystalline bundles of polysaccharides that consist of thousands of linked glucose molecules. Chains of sugar molecules are also found in hemicellulose, it is however an amorphous substance. More particularly, hemicellulose consists of a random combination of various carbohydrate molecules such as xylose, mannose and arabinose. Lignin, a macromolecule of substituted phenols, binds together the other components.

There are known ways of converting cellulose and hemicellulose into fuels and chemicals. The most common technique is to submit these to hydrolysis (using acid catalysts or enzymes) breaking these into their constituent sugars: the resulting molecules (mainly glucose) are fermented in ethanol (or other chemical intermediates) in the presence of yeasts. For example, bioethanol is currently produced by enzymatic fermentation of sugars from various biomass sources such as corn-grain (U.S.A.) or sugar cane (Brazil).

Lignin is much more difficult to depolymerize or decompose into its constituents. It is only possible to decompose lignin in quite harsh and hydrogenating conditions because of known its high oxygen content and the dominance of aromatic structures.

In recent years, environmental and social considerations have led to the use of new raw materials. In fact, all around the world, it is stressed that new biomass conversion technologies must not compete with food production, as first-generation biofuels do. Biodiesel, for example, is currently derived from rapeseed (canola) or soya bean oil while bioethanol is currently produced from plant matter containing starch or sugar. These processes compete with the use of these materials as sources of food.

Second-generation biofuels and biochemicals (those that do not directly compete with use as food), are derived from cellulose-hemicellulose based matter. Levulinic acid (reference 1), formic acid and their alkyl esters (ex. ethyl levulinate and ethyl formate, respectively) belong to such category of bio-fuels and biochemicals. In contrast, more advanced generation biofuels like those of the present invention are derived from all the three main components of the biomass, e.g. including lignin.

### Production of alkyl levulinates and light alkyl esters

The catalytic conversion of cellulose and hemicellulose into alkyl levulinates and light alkyl esters is known to be carried out in a) a single step process or b) a two- step process.

In a single step process, alcohol is used as a reactant and solvent. At least one acidic catalyst is used, typically a mineral acid diluted in the alcohol. The final products of the reaction consist mainly of alkyl levulinate (main product), levulinic acid, alkyl formate, 2-furfural (2-furfuraldehyde), alkyl acetate and solid "residues". The use of alcohol allows the occurrence of two chemical reactions: alcoholysis and esterification. However, the by-product dialkyl ether is also produced directly from the alcohol in quite significant amounts, the amounts varying with process conditions such as temperature. If the alcohol is ethanol, this ether is diethyl ether (ethyl ether). Diethyl ether can be considered, because of its high volatility at room temperature, as an inconvenience for various operations (handling, storage). Solid residues (commonly called lignin char) are also produced in a significant quantity.

It is to note that lignin char is a complex mixture of solid polymeric and resinous products formed by side-conversion (degradation-condensation) of various reaction intermediates from cellulose and hemicellulose. Lignin char that is present in the final suspension in both dissolved and (mainly) solid forms includes also the unconverted lignin (most of the time, seriously degraded).

In a two-step process, both catalytic steps involve acidic catalysts. The first step is the hydrolysis of cellulose and hemicellulose: this reaction produces levulinic acid and some by-products such as formic acid and 2-furfural. The second step is the esterification of the resulting acids, producing the corresponding alkyl esters. It is to note that, in some harsh conditions, 2-furfural resulting from the acid catalyzed degradation of the reaction intermediates of hemicelluloses, is converted into formic acid.

It is also noted that in the step of hydrolysis of cellulose and hemicellulose of the lignocellulosic material, there is a complex sequence of thermochemical and catalytic events: aperture of the lignocellulosic biomass structure, exposure of the cellulose and hemicellulose components, catalytic decrystallization/depolymerisation of cellulose and catalytic depolymerisation of the amorphous hemicellulose into respective sugar molecules, and finally, dehydration-decomposition of the latter into levulinic acid and formic acid, and 2-furfural, respectively. All these physico-chemical changes occur in the presence of a diluted solution of mineral acid and at moderately elevated temperatures. Usually, once the hydrolysis is completed, it is necessary to extract the produced levulinic acid from the lignin chars by various extractive techniques. Finally, at the end of the second step (esterification), ethyl levulinate has to be separated from other by-products.

### Extraction-separation of the final products

In the prior art, several problems arise with the extraction and then the separation of the products being produced in the reaction phase.

The important mass of solid residues (tars or lignin char) that usually act as a sponge for the liquid products, needs to be separated using filtration, centrifugation, etc. These techniques normally lead to important losses of products and can be energy consuming without resulting in sufficiently char-free liquid mixtures (of products) because some char are still dissolved in these aqueous or alcoholic mixtures.

Distillation (fractional distillation), vacuum-distillation, evaporating-stripping, solvent extraction, etc., are separation-purification techniques that are quite demanding in energy, and/or that can make use of harmful solvents.

Therefore, the contribution to the production cost of these conventional techniques of products extraction-separation can be enormous (typically larger than 60 %).

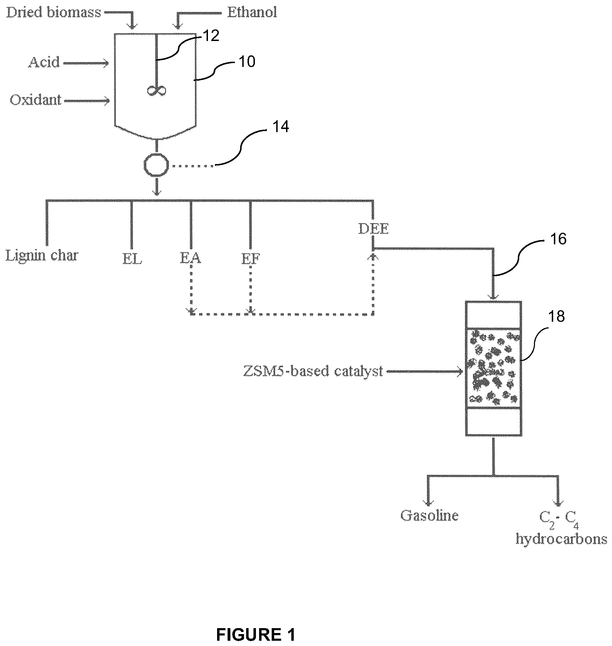

Recently, a fully integrated apparatus has been developed that allows production of alkyl levulinates and related liquid products from cellulosic biomass and to carry out the extraction-separation of these products by using appropriate procedures. This one-pot system, described in reference 2 (R. Le Van Mao, Q. Zhao, G. Dima and D. Petraccone, Catalysis Letters (2011) 141: 271-276), consists of a batch reactor connected to a system of condensers, that are in turn connected to a dry-vacuum system (DVS). The latter device can deliver a mild vacuum up to a maximum of 3-5 torr (see Figure 1 of reference 2). No environmentally harmful solvent is used and the obtained liquid fraction can be directly blended after drying into gasoline or diesel/biodiesel. The entire system used for the extraction-separation of the final products is named MVAD or mild vacuum-assisted distillation.

With such experimental set-up, it is possible to carry out the conversion of cellulosic biomass into alkyl levulinates and related liquid products by two alternative procedures (reference 2):
a) the direct method (D) consists of performing the conversion in acidic medium and with ethanol that acts as co-reactant and solvent;
b) the sequential method (SEQ) consists of first carrying out the acid hydrolysis of the cellulose (and hemicellulose) of the biomass, producing levulinic acid and other carboxylic acids. Water of the reaction medium is then removed by using the MVAD procedure, and is replaced by ethanol that converts these acids into ethyl esters. In such sequential procedure, the liquid acid catalyst is used for both steps.

The extraction-separation of the final products is the same for both procedures, D and SEQ.

Reference 2 reports that the yields in alkyl levulinates (particularly, ethyl levulinate) were almost the same for the two procedures. However, it was found later that the two-step procedure (SEQ) is too energy consuming whereas the direct method (procedure D) produced diethyl ether (DEE) as a by-product (by direct dehydration of ethanol) that might be significant at the temperatures used (180°C - 200°C).

### Production of diethyl ether (DEE)

DEE is the unwanted by-product because of its low boiling point (+ 34.5°C), its relatively high flammability and its quite limited commercial use (mainly as an organic solvent). Thus, limited production of DEE or its near total elimination during biomass conversion is highly recommended.

### Raw materials used

Reference 2 shows that several raw biomass materials were converted to biofuels or biochemicals, for example: wood chips (jack pine, spruce,...), paper pulp, switch grass, forestry residues and municipal wastes. It is obvious that their individual composition in cellulose, hemicellulose, lignin and other species, is different in accordance with the type of cellulosic biomass material being used. Therefore the resulting product spectrum depends on the composition of the raw material used.

### Lignin

In the prior art regarding the acid-treatment of biomass, lignin is typically slightly depolymerised so that cellulose and hemicellulose can be released for further conversion to liquid biofuels or biochemicals. In the prior art, the lignin itself essentially did not convert to biofuels or biochemicals.

References 1-7 are examples of prior art technology.

### SUMMARY OF THE INVENTION

The present invention relates to a method/procedure in two steps both involving catalysts for conversion of ligno-cellulosic biomass into biofuels and biochemicals.

The first step converts ligno-cellulosic biomass materials into liquid products and a solid residue called tars or lignin char. This consists of submitting said materials to a cracking reaction in oxidative/ acidic/ ethanol containing/ medium at moderate temperature.

The second step can essentially eliminates the unwanted by-product DEE by conversion over acidic nanocatalyst, preferably zeolite, most preferably ZSM-5 zeolite. Each step will be described in further detail.

In relation to the first step, the ligno-cellulosic biomass materials suitable for use by the present invention include all biomass materials that contain cellulose, hemicellulose and lignin. The preferred catalyst is sulphuric acid present in very dilute solution in ethanol. The preferred oxidizing species is hydrogen peroxide in aqueous solution. Most preferred oxidation activating species include Fe (II) preferred oxidation species include Fe (II) ions, Ti (IV) ions, H₂MoO₄ and/or methyltrioxorhenium (VII), these species being added to the reaction medium in very small amounts. The combination of Fe (II) ions and hydrogen peroxide is known as Fenton's reagent. By using an inorganic/organic sulphite or carbonate that can bind to furfuraldehyde-based intermediates, thus decreasing the rate of polymerization of the latter species, better yields of wanted products are obtained.

The moderate temperature is in the range of about 120°C to about 230°C.. Whenever used herein, "about" means + or - 10 % of the values. It is seen that a multi-heating-step procedure is preferably used to increase product yields.

The main conversion products of the ligno-cellulosic biomass are liquid ethyl esters such as ethyl levulinate, ethyl formate and ethyl acetate. Some by-products are also obtained (methanol, 2-furfural, succinic acid, levulinic acid), however, in smaller amounts.

The second step can essentially eliminate DEE by conversion over acidic nanocatalyst, preferably zeolite, most preferably ZSM-5 zeolite to obtain gaseous hydrocarbons and gasoline grade liquids. The DEE conversion can be carried out in a tubular reactor. The temperature ranges from about 280°C to about 340°C. Gaseous products include C₂-C₄ olefins, diolefins and paraffins. Liquid hydrocarbons are in the gasoline range, containing a large proportion of BTX (benzene, toluene and xylenes) aromatics. A moderate dilution of DEE with water was seen to considerably decrease the coke deposition onto the zeolite surface, thus contributing to increasing the on-stream stability of the catalyst (decreasing thus the need for frequent catalyst regeneration, i.e. decreasing the emission of greenhouse gases, carbon oxides). Finally, if other light ethyl esters and methanol produced in the first steps are fed to the second reactor along with DEE, essentially only hydrocarbons are obtained in corresponding yields, the various oxygenates in the feed being essentially converted.

### BRIEF DESCRIPTION OF FIGURE 1

Figure 1 is schematic representation of a production plant based on the present invention.

### DETAILED DESCRIPTION OF THE INVENTION

Other objects, advantages and features of the present invention will become more apparent upon reading of the following description of specific embodiments thereof, given by way of examples.

### Conversion of biomass into ethyl esters

The present invention is illustrated in further details by the following non-limiting examples of the production of ethyl esters and their extraction-separation from the conversion medium.

### Experimental

Referring to Figure 1, a Parr pressure reactor (10) (Capacity = 1 liter) equipped with a magnetic-driven stirrer, a water cooling system, temperature and pressure gauges, and safety devices was used for our testing. As reported in reference 1, this reactor was connected with a series of condensers (14) and a dry-vacuum system (Welch, Model 2028) that could provide a mild vacuum from 1 atmosphere to 2-3 torr. An assembly of collecting flasks and valves (not shown) allowed the collection of liquid products during the run without disturbing the vacuum assisted distillation operation.

A gas chromatograph set (Agilent Technologies 7890 A, Network GC system) equipped with a DB-Wax capillary column (always from Agilent Tech), was used for the analysis of the reaction products (ref. 1). 1-propanol was used as internal standard because water was always present in the liquid products.

In the presence of ethanol and dilute solution of sulphuric acid, the reaction products are the following:

Ethyl levulinate (EL), levulinic acid (LA), ethyl formate (EF), ethyl acetate (EA), others: 2-furfural (2-F) and eventually, methanol (MeOH). RP is the sum of all these products given by the biomass materials.

Diethyl ether (DEE) is formed directly from ethanol in presence of the mineral acid and is considered a by-product.

### Raw cellulosic materials used

Results obtained with three biomass materials are herein reported, that include:
a) Wood chips JP (jack pine of Quebec). This ligno-cellulosic material has the following approximate composition (dried form): cellulose = 42 wt %, hemicellulose = 26 wt % , lignin = 31 wt % and others = 1 wt %;
b) Wood chips SP (spruce of Quebec). This ligno-cellulosic material has the following approximate composition (dried form): cellulose = 40 wt %, hemicellulose = 27 wt %, lignin = 32 wt % and others = 1 wt %. It is to note that the chemical structures of the hemicelluloses of the wood JP and SP are different from each other;
c) Bleached paper pulp (provided by Cascades Corporation) was used as a model of cellulosic biomass materials. This paper pulp had high contents in cellulose and hemicellulose (78 wt % of cellulose, 16 wt % of hemicellulose and the balance (6 wt %), being some organic and inorganic compounds). Before the experimentation, the paper pulp was cut in small pieces (size of a few cm) and dried at 120°C overnight (average moisture content = 6.7 wt %).

### Coupling of catalytic/chemical reactions: Use of an acid catalyst and a chemical oxidizer

Example 0 (Table 1) reports the yields of products obtained by the acidic conversion of wood chips (jack pine) in ethanol medium. Example 1 reports the results that were obtained with a reaction medium containing H₂O₂ (oxidizer), there are some large differences in product yields:
- Ethyl levulinate is produced at significantly higher yield,
- The yields of ethyl formate and ethyl acetate drastically increased, suggesting a strong action of the oxidizer on reaction intermediates, mostly on those of hemicellulose,
- Methanol was formed in quite significant amount, suggesting that lignin (very) partially underwent conversion in such oxidizing-acidic medium,
- In oxidizing conditions used in examples 1 to 6, almost no levulinic acid was obtained, meaning that the esterification of levulinic acid was complete. In addition, 2-furfural disappeared almost completely from the product spectrum, suggesting that this compound normally formed from hemicellulose was further converted into formic acid (finally, ethyl formate by reaction with ethanol).

Table 1 - Effect of the oxidizer in the reaction medium and a pre-treatment phase in the heating procedure on the product yields.

Mass of raw material: wood chips (jack pine, dried) = 80 g; 45 g of 3.0 N H₂SO₄ in absolute ethanol, 210 g of absolute ethanol, 25 g of H₂O₂ (40 wt %): thus, acid concentration in the liquid phase = 2.60 wt %, concentration of H₂O₂ = 3.57 wt %.

| **Example number** | **0(*)** | **1** | **2** | **3** | **4** | **5** | **6** |
|---|---|---|---|---|---|---|---|
| H₂O₂ | NO | YES | YES | YES | YES | YES | YES |

| *Heating steps* | | | | | | | |
|---|---|---|---|---|---|---|---|
| T₁ (°C)/time in min | NO | NO | 140/30 | 140/60 | 140/120 | 140/120 | 140/60 |
| T₂ (°C)/time in min | 190/100 | 157/180 | 157/180 | 157/180 | 157/180 | 157/120 | 153/180 |

| *Product yield (wt%)* | | | | | | | |
|---|---|---|---|---|---|---|---|
| EL (**) | 15.8 | 14.6 | 20.3 | 21.3 | 21.5 | 16.3 | 18.2 |
| EF | 5.4 | 13.1 | 15.7 | 16.0 | 15.0 | 15.7 | 15.3 |
| EA | 2.6 | 6.2 | 6.2 | 6.5 | 5.7 | 6.1 | 6.3 |
| 2-F | 0.9 | 0.0 | 0.3 | 0.0 | 0.3 | 0.4 | 0.3 |
| Methanol (others) | 0.0 (2.2) | 1.5 | 1.4 | 1.2 | 1.3 | 1.4 | 1.4 |
| Total Reaction products (RP) (sum of five above lines) | 26.9 | 35.4 | 43.9 | 45.0 | 43.8 | 39.9 | 41.5 |
| DEE (***) as by-product | 64.3 | 46.2 | 47.3 | 47.8 | 47.6 | 44.0 | 41.5 |

| | | | | | | | |
|---|---|---|---|---|---|---|---|
| (*) Results reported in reference 1 in operating conditions D with no H₂O₂ in reaction medium. (**) No or very little LA (< 0.1 wt %), produced. (***) DEE is the product of the direct dehydration of ethanol. | | | | | | | |

### Effect of the pre-conditioning (partial delignification) step on the product yields

When a first heating step was incorporated into the conversion procedure (examples 2 to 6 of Table 1), the yields of ethyl levulinate and ethyl formate dramatically increased, suggesting that the cellulose was better exposed for conversion into esters of its levulinic and formic acids. These examples (2-6) also showed that there was a certain balance between the pre-conditioning phase and the main step, so that a maximum yield of products could be obtained (conditions of example 3). To support the hypothesis of a low-temperature pre-conditioning phase having positive effect, we can evoke the boiling point of hydrogen peroxide that is 150°C: in fact, it was suggested that the protonated form of H₂O₂ could be a strong oxidizing agent for carbohydrates [reference 2].

### Ligno-cellulosic versus cellulosic materials

In order to show the differences in the product spectrum when a cellulosic material such as paper pulp was used instead of a ligno-cellulosic material such as jack pine chips, paper pulp that contain little or no lignin was tested in the same conditions as example 3 of Table 1 (wood chips).

Results of Example 7 of Table 2, when compared with those of Example 3 of Table 1 shows that:
a) Paper pulp did not produce any methanol because the latter should come from the lignin;
a) The production of ethyl levulinate (and also levulinic acid) should be higher with the paper pulp because the latter raw material contained much more cellulose than the wood chips.

Because the degree of crystallinity of cellulose in the paper pulp was higher than that of the wood chips, slightly more severe conditions of conversion (slightly higher acid concentration and reaction temperatures) were used with Example 8 (Table 2): effectively, the conversion to ethyl levulinate was higher suggesting that the new operating conditions could increase the efficiency of the acid attack of the cellulose in the paper pulp.

On the other hand, by using a one-step conversion procedure (Example 9 versus Example 8, all of Table 2), similar levels of conversion into ethyl levulinate and other esters were obtained, suggesting that the first conversion step may be skipped when the used cellulosic biomass like the paper pulp did not contain lignin.

Thus, data of Tables 1 and 2 indicate that the lignin component was also converted with an acidic and oxidative medium (presence of methanol, known to be produced by decomposition of lignin). However, the level of conversion of lignin was still low.

**Table 2 - Conversion of paper pulp, an example of cellulosic material.**

| **Example number** | **7** | **8** | **9** |
|---|---|---|---|
| Acid concentration (wt %) | 2.60 | 2.75 | 2.75 |
| Concentration of H₂O₂ (wt %) | 3.57 | 3.53 | 3.53 |

| Temperature in °C (time in min) | | | |
|---|---|---|---|
| Step 1 | 140 (60) | 143 (60) | NO |
| Step 2 | 157 (180) | 160 (180) | 163 (180) |

| *Product yields (wt %)* | | | |
|---|---|---|---|
| EL + LA | 23.9 | 30.2 | 31.2 |
| EF | 17.6 | 20.6 | 17.9 |
| EA | 5.2 | 5.1 | 4.6 |
| 2-F | 1.2 | 0.0 | 0.0 |
| Methanol | 0.0 | 0.0 | 0.0 |
| Total RP (sum of five above lines) | 47.9 | 55.9 | 53.7 |
| DEE(by-product) | 31.9 | 50.3 | 45.5 |

### Effect of the content of hydrogen peroxide

Hydrogen peroxide is a strong oxidizer for carbohydrates and their reaction intermediates. Because the various organic acids formed are degradation products of theses carbohydrates, hydrogen peroxide has a strong influence on the distribution of the final products. It is worth noting that with the increased concentration of the oxidizer, the yields of methanol and other light ethyl ethers noticeably increase at the expenses of ethyl levulinate.

**Table 3 - Effect of the concentration of H₂O₂ in the liquid phase. Acid concentration = (2.60 wt %, water = 5.4 wt %, heating steps = same as in Example 3).**

| **Example number** | **10 (=Ex. 3)** | **11** | **12** |
|---|---|---|---|
| Concentration of H₂O₂ (wt%) | 3.57 | 5.36 | 1.79 |
| EL | 21.3 | 17.8 | 17.5 |
| LA | 0.0 | 1.1 | 1.0 |
| EF | 15.7 | 17.5 | 16.4 |
| EA | 6.2 | 6.8 | 5.4 |
| 2-F | 0.0 | 0.4 | 0.3 |
| Methanol | 1.2 | 1.7 | 1.1 |
| Total RP (sum of five above lines) | 45.0 | 45.3 | 41.7 |
| DEE (by-product) | 47.8 | 45.5 | 51.0 |

### Presence of Fe (II) - based catalyst in the reaction medium (use of Fenton-type reagents)

Fe(II) ions, added to H₂O₂, is known to form the Fenton's reagent with strong oxidizing properties for converting numerous organic compounds [reference 5]. Fenton-type reagents are usually used to degrade organic contaminants in waters. Surprisingly, in the cellulosic biomass conversion, Fe(II) ions when incorporated into the reaction medium that already contains hydrogen peroxide, significantly increased the yield of all ethyl esters, particularly the ethyl levulinate, as reported in Table 4.

**Table 4 - Effect of Fenton's reagent (Fe(II) + H₂O₂) in the reaction medium.**

| **Example number** | **13** | **14** | **15** | **16** |
|---|---|---|---|---|
| [Fe(II)/H₂O₂] x 10⁻² [(g/g) (mol ratio)](*) | 0.0/0.0 | 0.4/0.25 | 0.8 / 0.49 | 1.6/0.97 |

| *Product yield (wt %)* | | | | |
|---|---|---|---|---|
| EL | 20.3 | 25.7 | 26.5 | 25.9 |
| LA | 1.5 | 1.6 | 1.8 | 1.6 |
| EF | 17.1 | 19.5 | 20.8 | 20.7 |
| EA | 5.1 | 6.1 | 7.5 | 6.4 |
| 2-F | 0.3 | 0.5 | 0.0 | 0.3 |
| Methanol | 1.6 | 1.6 | 1.4 | 1.6 |
| Total RP (sum of five above lines) | 45.9 | 55.0 | 58.0 | 56.5 |
| DEE (by-product) | 51.6 | 47.1 | 54.4 | 60.7 |

| | | | | |
|---|---|---|---|---|
| (*) Ferrous sulphate heptahydrate (Anachemia). Concentration: Acid = 2.60 wt %, H₂O₂ = 4.39 wt %. | | | | |

Fe (II) ions present in quite small amounts produce hydroxyl radicals with hydrogen peroxide that contribute to a more powerful and selective oxidizing cracking of the furfuraldehyde functions of the reaction intermediates in the cellulosic biomass conversion.

Ti (IV) ions in the presence of hydrogen peroxide is a powerful oxidizing catalyst, Ti (IV) ions, in the form of Ti (IV) oxysulfate-sulphuric acid or Ti (IV) ethoxide, can be incorporated in very small amounts into the reaction medium that already contains the mineral acid, hydrogen peroxide and ethanol. H₂MoO₄ and methyltrioxorhenium (VII) can have similar effect.

Example 20 of Table 5, paragraph [0061] shows the positive effect of Ti (IV) on the yield of ethyl levulinate.

It is worth noting that hydrogen peroxide or the Fenton's reagent is used to degrade (very partially) the lignin component in the pre-treatment of ligno-cellulosic materials. This pre-treatment is necessary for "opening" the wood or biomass structure, so that chemical reactants or enzymes can reach the other components: hemi-cellulose and cellulose. This invention advantageously uses hydrogen peroxide, Fenton's reagent or hydrogen peroxide + TiO₂ also to selectively oxidize cellulose and hemi-cellulose: surprisingly, their degradation into carboxylic acids much better occurs that results in very significant improvement of the yields of the final products, i.e. ethyl esters (Tables 1 and 4).

### De-coupling of reaction networks

Polymerization of furfural and other aldehydic intermediates produced by the conversion of ligno-cellulosic biomass generally accompanies the production of various liquid products, the commercially valuable carboxylic acids (then rapidly convert into ethyl esters in the presence of ethanol) and others. These polymers form with the unconverted lignin the solid tars or lignin char (case of lignin containing biomass).It is usually very difficult to decrease the rate of formation of these solid tars.

Sodium sulphite and other inorganic or organic sulphites as well as sodium and calcium carbonates are known to bind to the aldehyde function of organic compounds. This invention can incorporate sodium sulphite (for instance) to the reaction medium, so that the rate of polymerization of various aldehydes intermediates or products can be lowered (inhibition of polymerisation). Thus, per compensation effect, the yields of other conversion products can be increased. Tables 5 and 6 report the results of such novel procedure: network 1 = conversion to liquid products, network 2 = polymerisation of aldehyde-based intermediates to tars. Data of Table 5 show that, effectively, network 2 is significantly depressed while network 1 is favoured, if sodium sulphite is used as inhibitor of polymerisation.

**Table 5 - Inhibiting the polymerization of furfural and other aldehyde-type reaction intermediates by incorporation of sodium sulphite. Reaction conditions: 140°C for 60 min and 157°C for 240 min; acid = 2.60 wt %, H₂O₂ = 3.57 wt %, [Fe(II)/H₂O₂] x 10⁻² (g /g /mol ratio) = 0.8/0.49.**

| **Example number** | **17** | **18** | **19** | **20*** |
|---|---|---|---|---|
| Na sulphite (wt %) | 0.4 | 0.5 | 0.7 | 0.5 |
| Acid concentration (wt %) | 2.6 | 2.8 | 2.8 | 2.9 |

| *Product yield (wt %)* | | | | |
|---|---|---|---|---|
| EL | 31.3 | 37.2 | 35.8 | 40.8 |
| LA | 2.7 | 4.5 | 5.4 | 4.1 |
| EF | 19.4 | 19.9 | 16.7 | 13.7 |
| EA | 6.3 | 5.9 | 5.2 | 5.4 |
| 2-F | 0.7 | 1.4 | 1.0 | 1.1 |
| Methanol | 1.6 | 1.5 | 1.5 | 3.2 |
| Total RP (sum of five above lines) | 62.0 | 70.4 | 65.6 | 68.2 |
| DEE (by-product) | 60.5 | 43.1 | 47.8 | 26.0 |

| | | | | |
|---|---|---|---|---|
| (*) Example 20: Use of Ti (IV) oxysulfate instead of Fe (II) sulphate with concentration of TiO₂ = ca. 0.03 wt %. | | | | |

In order to decrease the production of DEE, quite low concentration of acid is used (1.2 wt %) and some water is also added (ethanol/water wt ratio = 3.8). The other parameters are = raw material = dried spruce chips, H₂O₂ = 2.8 wt %, main catalyst: [Fe(II)/H₂O₂] x 10⁻² (g /g /mol ratio) = 0.8/0.49. The reaction temperatures are as follows: first heating step = 195 °C for 12 minutes, and second heating step = 172 °C for 35 ° C.

Table 6 shows that by using polymerization inhibitor (sodium sulphite, sodium carbonate-decahydrate, sulphurous acid ions, para-toluene sulfonic acid monohydrate (PTSA), calcium carbonate, by using very low concentration of acidic catalyst (sulphuric acid) and by adjusting the reaction temperatures and times, it is possible to significantly decrease the formation of DEE while the yields of the main products are kept almost unchanged, except for the levulinic acid that significantly increases.

| **Example number** | **21** | **22** | **23** | **24** | **25** |
|---|---|---|---|---|---|
| Polymerization inhibitor | Na₂SO₃ | Na₂CO₃ | H₂SO₃ | PTSA | CaCO₃ |

| *Product yields(wt %)* | | | | | |
|---|---|---|---|---|---|
| EL | 30.1 | 30.2 | 40.5 | 40.0 | 31.2 |
| LA | 8.7 | 8.8 | 8.9 | 9.8 | 8.4 |
| EF | 17.6 | 17.3 | 21.2 | 17.6 | 18.1 |
| EA | 8.4 | 8.7 | 9.6 | 7.4 | 8.0 |
| 2-F | 4.5 | 4.2 | 5.5 | 4.2 | 4.4 |
| Methanol | 1.0 | 2.1 | 1.7 | 1.4 | 1.2 |
| Succinic acid | 2.3 | 4.4 | 4.0 | 3.2 | 2.8 |
| Total RP (sum of five above lines) | 72.6 | 75.7 | 91.4 | 83.6 | 74.1 |
| DEE (by-product) | 16.5 | 13.2 | 17.1 | 15.6 | 17.6 |

Close observation of these results shows that a small concentration of H₂SO₃ or PTSA (< 0.2 wt %) can significantly enhance the yield of reaction products (RP) formed from the biomass itself (Ex. 23 and 24).

### Conversion of (unwanted by-product) diethyl ether (DEE) into commercially valuable hydrocarbons

The present invention is illustrated in further details by the following non-limiting examples of the production of hydrocarbons from DEE, the main by-product of the conversion of biomass into ethyl esters. In fact, DEE and, optionally, light ethyl esters being produced by the acid ethanolysis of biomass material, are sent over a zeolite acid catalyst, preferably ZSM-5 zeolite (acid form). The products of this catalytic reaction are hydrocarbons containing from 1 to 12 carbon atoms. In particular, liquid hydrocarbons having a number of carbon atoms ranging from 5 to 12 are those that normally correspond to petroleum gasoline. The presence of aromatics (BTX) enhance the octane rating of such gasoline.

### Experimental

Referring to Figure 1, DEE (in some run, DEE and light ethyl esters) was injected by mean of an infusion pump into a vaporizer- vapour mixer (16). The resulting vapour was then sent into a tubular reactor (18) (quartz tube, 50 cm long, 1.5 cm in outer diameter and 1.2 cm in inner diameter). The temperatures were controlled and regulated by automatic devices (not shown) that were connected to chromel-alumel thermocouples (set in the catalytic bed and in the pre-heating zone) and the heating furnace.

The testing conditions were as follows: temperature = 280, 300 and 320°C ± 2°C; weight hourly space velocity (WHSV): 1.5 - 7.5 h⁻¹, weight of catalyst = 1.0-2.0 g, run time = 3h. In some runs, water was added to the feed, using another infusion pump being connected to the vaporizer-gas mixer.

A ZSM-5 based catalyst was used in the form of extrudates (H-ZSM-5, Si/Al = 50, 20 wt % binder).

Liquid and gaseous products were collected separately, using a system of condensers. The gas-phase components were analyzed using a FID gas chromatograph that was equipped with a 30m GS-capillary column (Agilent J & W Scientific), while the analysis of the liquid phase was performed using another FID gas chromatograph equipped with a HP-5 capillary column (Agilent J & W Scientific, 30 m). The liquid phase was also analyzed using a FID gas chromatograph equipped with a DB-Wax capillary column (Agilent).

### Catalytic conversion of diethyl ether (DEE) into hydrocarbons over ZSM-5 zeolite

Diethyl ether, by-product of this process, is then sent into a tubular reactor that is heated at 300°C. Reaction conditions are reported in Table 7. The conversion of DEE is almost complete. It is seen that the dilution of DEE by water is extremely beneficial because the coke deposition dramatically decreases when steam is present, so that the zeolite catalyst can be used for quite long time without any need for regeneration (operation that consists of coke removal by combustion). The liquid hydrocarbons, having a boiling point ranging from C₅ to C₁₁, can be considered as gasoline-grade liquid, having a high octanerating because its relatively high content in BTX aromatics. The production of methane is almost nil: this is really an advantage because methane is not a commercially very valuable product.

Not only the DEE can be used, but also a mixture containing DEE and some low ethyl esters such as ethyl formate or ethyl acetate, can also be converted into hydrocarbons. Finally, the separation of the products of this catalytic reaction (hydrocarbons-water) is an easy operation (simple decantation).

It should be noted that instead of using a tubular reactor working under atmospheric pressure, other reaction systems can be utilized that may give higher yields of liquid products (ex: pressure tubular reactor). Other reactor shapes can also be used.

**Table 7 - Conversion of product diethyl ether (DEE) into hydrocarbons over ZSM-5 zeolite (Temperature = 300°C; weight of catalyst = 2 g).**

| **Example number** | **26** | **27** | **28** | **29** |
|---|---|---|---|---|
| WHSV (h⁻¹) | 3.0 | 1.5 | 1.5 | 1.5 |
| H₂O/DEE weight ratio | 0 | 0 | 0.5 | 1.0 |
| Total conversion (wt %) | 97.7 | 98.4 | 98.2 | 98.2 |

| *Product yield (wt %):* | | | | |
|---|---|---|---|---|
| Liquid hydrocarbons (BTX aromatics) | 53.3 (30.7) | 54.7 (28.2) | 56.2 (25.7) | 54.5 (21.7) |
| C₂-C₄ paraffins | 37.0 | 40.0 | 34.7 | 26.8 |
| Methane | 0.03 | 0.02 | 0.02 | 0.01 |
| C₂-C₄ olefins and diolefins | 6.5 | 2.2 | 6.8 | 16.9(*) |
| Coke | 0.8 | 1.5 | 0.5 | < 0.1 |

| | | | | |
|---|---|---|---|---|
| (*) Ethylene = 6.2 wt %, Propylene = 4.8 wt %, butenes and butadienes = 5.9 wt %. | | | | |

As reported in Table 7, at the temperature tested, the conversion of DEE is essentially complete. The yield in liquid hydrocarbons (gasoline-grade), even under atmospheric pressure, is much higher than 50 wt %. Such gasoline has a high octane rating because its BTX aromatics content is relatively high. C₂-C₄ olefins and paraffins are commercially valuable hydrocarbons owing to their uses in the petrochemical industry (production of important plastics, synthetic fibers and rubbers).

It is worth noting that:
a) The yield of methane is low (< 0.1 wt %);
b) The production of coke (main cause of catalyst fouling that may result in quite rapid activity decay) is relatively low (examples 26 and 27). Moreover, the addition of water (steam) to the DEE feed (examples 28 and 29 versus example 27) does not decrease the total conversion although some changes are observed in the composition of products. The most surprising result is that the coke deposition drastically decreases: this means that the on-stream stability of the catalyst is considerably improved, leading to improved economics for the process (less catalyst regeneration operations needed) and also importantly, much lower emission of greenhouse gases (carbon oxides);
c) Several catalysts have been tested, that include various zeolites (H-Y, US-HY, acidic mordenite) as well as amorphous acidic alumina. The preferred catalyst in terms of activity (conversion) and high yields of gasoline (and light hydrocarbons) is the H-ZSM-5. Even with the same zeolite structure, the H-ZSM-5 (Si/Al = 50) is most preferred over the H-ZSM-5 (Si/Al = 25) or the H-ZSM-5 (Si/Al = 100). Still preferably, modifying the ZSM-5 zeolite with Zn or Ga results in higher product BTX (aromatics) yield.

### Catalytic conversion of light product liquid into hydrocarbons over ZSM-5 zeolite

As an option, the light products normally obtained in the first conversion step (DEE along with other light ethyl esters and some methanol, see examples 2 to 6) in ethanol solution, herein called light fraction LF, were sent to the catalytic reactor that contained the H-ZSM5 (50). Table 8 shows that all these liquid products were completely converted into hydrocarbons.

**Table 8 - Conversion of liquid fraction F3 into hydrocarbons over ZSM-5 zeolite catalyst**

| **Example number** | | **30** | **31** |
|---|---|---|---|
| Reaction temperature (°C) | | 300 | 320 |
| Water added (wt %) | | 0 | 20 |
| Composition/yields (wt %) | Light fraction (LF) | | |
| Diethyl ether (DEE) | 38.9 | trace | Trace |
| Ethyl formate (EF) | 7.4 | trace | Trace |
| Ethyl acetate (EA) | 3.2 | trace | Trace |
| Methanol (MeOH) | 0.4 | trace | Trace |
| Ethanol (EtOH) | 50.1 | 0.3 | 0.9 |
| Methane | 0 | < 0.1 | < 0.1 |
| C₂-C₄ paraffins | 0 | 24.8 | 39.4 |
| C₂-C₄ olefins | 0 | 26.5(*) | 15.7(**) |
| Gasoline-grade hydrocarbons (BTX aromatics) | 0(0) | 46.6 (9.5) | 43.5 (7.7) |
| Coke | 0 | 1.8 | 0.4 |

| | | | |
|---|---|---|---|
| (*) Ethylene = 14.5 wt %, propylene = 4.8 wt %, butenes and butadienes = 7.2 wt %. (**) Ethylene = 7.4 wt %, propylene = 3.9 wt %, butenes and butadienes = 4.4 wt %. | | | |

### Various combinations of final products

Following figure shows schematically the technology of the present invention: the conversion of ligno-cellulosic materials can result in one of the following spectra of final products.
1) Ethyl levulinate + gasoline + C₂-C₄ hydrocarbons+ ethyl acetate + ethyl formate + methanol, if only DEE is sent to the second reactor.
2) Ethyl levulinate + gasoline (C₅-C₁₁ hydrocarbons) + C₂-C₄ hydrocarbons, if the light ethyl esters and methanol by-products are sent together with DEE in the second reactor.

Particularly interesting is option 2) for the commercial uses of its final products: diesel additive (ethyl levulinate), high octane rating gasoline, C₂-C₄ olefins and paraffins as intermediates (for polymers)/feedstocks for the petrochemical industry. In particular, C₂-C₄ paraffins (ethane and mostly propane and butanes) can be used as motor fuel (liquefied petroleum gas, LPG).

As reported in the examples of Tables 5 and 6, in the best conditions of the main reaction (Examples 20 to 25), by using the various catalytic effects combined in the reaction medium as mentioned earlier (oxidation by hydrogen peroxide/Fenton's reagent, and use of a polymerization inhibitor) the liquefaction of the ligno-cellulosic biomass can reach the level of 60 wt % of all the biomass used. A rough estimation shows that, in the case of jack pine or spruce wood chips used as raw material, almost all the cellulose component and up to 60-65 wt % of hemicellulose component are converted in liquid products (the rest of the hemicellulose being transformed in solid polymeric species probably through the 2-furfural). The presence of methanol and that of some short carboxylic acids such as formic and acetic acids (both esterified by ethanol), and also succinic acid, indicate that lignin is also converted in such reaction conditions, however, to a much lower extent (15 to 20 wt %). More lignin can be converted into liquid products if the reaction conditions are harsher (higher temperature, more oxidizer and longer digestion time). This is a good approach for increasing the liquefaction level of ligno-cellulosic materials, over 60 wt % with spruce or pine wood chips herein investigated. However, these newly formed products are a "mixture" of some water-soluble phenol derivatives that have probably lower commercial values.

The second step of the present invention that uses a ZSM-5 type zeolite catalyst to convert diethyl ether (DEE) into hydrocarbons (gasoline and other gaseous hydrocarbons), as reported in Table 7. DEE can also act as hydrogen donor when it is fed with other compounds such as the light esters as reported in Table 8. Preliminary testing of such zeolite-catalyzed reaction using a mixture of DEE and these phenolic products shows that we can obtain a gasoline that is richer in aromatics than when DEE is used alone.

### References

(1) S. W. Fitzpatrick, US Patent 5,608,105 (Mar. 4, 1997).
(2) R. Le Van Mao, Q. Zhao, G. Dima and D. Petraccone, Catal. Lett. 141 (2011) 271.
(3) Q. Xiang, Y.Y. Lee, Appl. Biochem Biotech 84-86 (2000) 153.
(4) M. Mascal, E.B. Nikitin, ChemSusChem 3 (2010) 1349.
(5) J.H. Merz, W.A. Waters, J. Chem.Soc. S15 (1949).
(6) R. Le Van Mao, L. Dufresne, US Patent 4,975,402 (Dec. 4, 1990).
(7) R. Le Van Mao, J. Yao, US Patent 5,135,898 (Aug. 4, 1992).

## Claims

1. A method for converting ligno-cellulosic biomass materials into ethyl esters and hydrocarbons, said method comprising the following steps: (a) the chemical-catalytic conversion of the biomass material into ethyl levulinate, light ethyl esters and other products including diethyl ether carried out with alcohol as reactant and solvent in dilute acidic medium and in the presence of an oxidizing agent, (b) the chemical-catalytic conversion of the diethyl ether and/or light ethyl esters into hydrocarbons by reaction with an acid nanocatalyst and (c) the recovery of the resulting products.

2. The method of claim 1 wherein the oxidizing agent is hydrogen peroxide.

3. The method of claim 1 wherein the oxidizing agent is a Fenton-type reagent.

4. The method of claim 1 wherein the oxidizing agent is hydrogen peroxide alone or hydrogen peroxide activated by Fe (II) ions and/or Ti (IV) ions.

5. The method of any one of claims 1 to 4 wherein the dilute acidic medium comprises an inhibitor of polymerization of aldehyde-type reaction intermediates, preferably sulfurous acid, para-toluene sulfonic acid and also, sodium sulfite, sodium carbonate, calcium carbonate.

6. The method of claim 1 wherein the acidic nano-catalyst is a zeolite of ZSM-5 type.

7. The method of claim 6 wherein the zeolite of ZSM-5 type has a Si/Al atom ratio ranging from 40 to 60.

8. The method of claim 6 wherein the ZSM-5 zeolite is modified by Zn or Ga.

9. The method of any one of claims 1 to 8 wherein the resulting products comprise ethyl levulinate (diesel-grade additive), light ethyl esters, preferably ethyl formate or ethyl acetate, methanol, gasoline-range hydrocarbons, C₂-C₄ hydrocarbons or mixtures thereof.

## Patentansprüche

1. Verfahren zum Umwandeln lignocellulosehaltiger Biomassematerialien in Ethylester und Kohlenwasserstoffe, wobei das Verfahren die folgenden Schritte umfasst: (a) die chemisch-katalytische Umwandlung der Biomassematerialien in Ethyllevulinat, leichte Ethylester und andere Diethylether umfassende Produkte, die mit Alkohol als Reaktionsmittel und Lösungsmittel in einem verdünnten sauren Medium und in Anwesenheit eines Oxidationsmittels durchgeführt wird, (b) die chemisch-katalytische Umwandlung des Diethylethers und/oder der leichten Ethylester in Kohlenwasserstoffe mittels Reaktion mit einem sauren Nanokatalysator und (c) Gewinnung der resultierenden Produkte.

2. Verfahren nach Anspruch 1, wobei das Oxidationsmittel Wasserstoffperoxid ist.

3. Verfahren nach Anspruch 1, wobei das Oxidationsmittel ein Fenton-artiges Reagens ist.

4. Verfahren nach Anspruch 1, wobei das Oxidationsmittel entweder Wasserstoffperoxid allein oder mittels Fe-(II)-Ionen und/oder Ti-(IV)-Ionen aktiviertes Wasserstoffperoxid ist.

5. Verfahren nach einem der Ansprüche 1 bis 4, wobei das verdünnte saure Medium einen Inhibitor der Polymerisation aldehydartiger Reaktionszwischenprodukte, vorzugsweise schwefelige Säure, para-Toluolsulfonsäure, und auch Natriumsulfit, Natriumcarbonat, Calciumcarbonat umfasst.

6. Verfahren nach Anspruch 1, wobei der saure Nanokatalysator ein Zeolith des ZSM-5-Typs ist.

7. Verfahren nach Anspruch 6, wobei der Zeolith des ZSM-5-Typs ein Si/Al-Atomverhältnis aufweist, das von 40 bis 60 reicht.

8. Verfahren nach Anspruch 6, wobei der ZSM-5-Zeolith mittels Zn oder Ga modifiziert ist.

9. Verfahren nach einem der Ansprüche 1 bis 8, wobei die resultierenden Produkte Ethyllevulinat (Dieselgüteadditiv), leichte Ethylester, vorzugsweise Ethylformat oder Ethylacetat, Methanol, benzingeeignete Kohlenwasserstoffe, C₂-C₄-Kohlenwasserstoffe oder Mischungen davon umfassen.

## Revendications

1. Procédé pour la conversion de biomasses ligno-cellulosiques en esters éthyliques et en hydrocarbures, ledit procédé comprenant les étapes suivantes : (a) la conversion catalytico-chimique de la biomasse en lévulinate d'éthyle, esters éthyliques légers et autres produits incluant de l'éther diéthylique, réalisée avec un alcool comme réactif et un solvant dans un milieu acide dilué et en présence d'un agent oxydant, (b) la conversion catalytico-chimique de l'éther diéthylique et/ou des esters éthyliques légers en hydrocarbures par réaction avec un nanocatalyseur acide et (c) la récupération des produits résultants.

2. Procédé selon la revendication 1, dans lequel l'agent oxydant est le peroxyde d'hydrogène.

3. Procédé selon la revendication 1, dans lequel l'agent oxydant est un réactif de type Fenton.

4. Procédé selon la revendication 1, dans lequel l'agent oxydant est le peroxyde d'hydrogène seul ou le peroxyde d'hydrogène activé par des ions Fe (II) et/ou des ions Ti (IV).

5. Procédé selon l'une quelconque des revendications 1 à 4, dans lequel le milieu acide dilué comprend un inhibiteur de polymérisation des intermédiaires de réaction de type aldéhyde, de préférence de l'acide sulfurique, de l'acide paratoluène sulfonique et également du sulfite de sodium, du carbonate de sodium, du carbonate de calcium.

6. Procédé selon la revendication 1, dans lequel le nanocatalyseur acide est une zéolithe de type ZSM-5.

7. Procédé selon la revendication 6, dans lequel la zéolithe de type ZSM-5 possède un rapport atomique Si/Al allant de 40 à 60.

8. Procédé selon la revendication 6, dans lequel la zéolithe ZSM-5 est modifiée par Zn ou Ga.

9. Procédé selon l'une quelconque des revendications 1 à 8, dans lequel les produits résultants comprennent du lévulinate d'éthyle (additif de qualité diesel), des esters éthyliques légers, de préférence du formiate d'éthyle ou de l'acétate d'éthyle, du méthanol, des hydrocarbures de la gamme gazole, des hydrocarbures en C₂-C₄ ou des mélanges de ceux-ci.
